# EUROPÄISCHE PATENTSCHRIFT

(11) **EP 0 690 133 B1**
(45) Veröffentlichungstag und Bekanntmachung des Hinweises auf die Patenterteilung: **10.10.2001**
(21) Anmeldenummer: 95109204.8
(22) Anmeldetag: 14.06.1995
(51) Int. Cl.: C12P 41/00, C07F 9/32, C07F 9/30, C12P 9/00

(54) **Verfahren zur enzymatischen Spaltung von 2-Amino-4-methylphosphinobutansäureamid-Derivaten**
Process for the enzymatic resolution of 2-amino-4-methyl phosphinobutanoic acid amides
Procédé de résolution enzymatique de dérivés d'amides d'acide amino-2-méthyl-4 phosphinobutanoique

(30) Priorität: 26.06.1994 DE 4422045
(43) Veröffentlichungstag der Anmeldung: 03.01.1996
(73) Patentinhaber: Aventis CropScience GmbH, 65929 Frankfurt am Main (DE)
(72) Erfinder: Willms, Lothar, Dr., D-65719 Hofheim (DE); Bartsch, Klaus, Dr., D-61462 Königstein (DE)

(56) Entgegenhaltungen:
- EP-A- 0 193 113
- EP-A- 0 346 658
- EP-A- 0 382 113
- EP-A- 0 382 114
- WO-A-89/10969
- BULL. CHEM. SOC. JPN. (BCSJA8,00092673);88; VOL.61 (10); PP.3699-704, RES. CENT. "KONSTR. POLYM.";SOFIA; 1528; BULG. (BG) Nachev I 'Organophosphorus analogs and derivatives of the natural L-amino carboxylic acids and peptides. I. Enzymic synthesis of D-, DL-, and L-phosphinothricin and their cyclic analogs'

## Beschreibung

L-2-Amino-4-methylphosphinylbutansäure - im folgenden als L-Phosphinothricin oder L-PTC bezeichnet - oder deren Salze mit Basen oder Säuren sind - wie aus der DE-A 2939269 bekannt geworden ist - die wirksame Komponente des chemisch leicht zugänglichen Racemats, der 2-Amino-4-methylphosphinylbutansäure. Gemäß Untersuchungen in der DE-A 2717440 besitzt L-Phosphinothricin eine ausgeprägte herbizide Wirksamkeit gegen ein breites Spektrum von Schadpflanzen, die D-Form ist inaktiv. Es erschien damit notwendig, ein brauchbares Verfahren zu entwickeln, durch das die L-Form in ökonomischer Weise zugänglich gemacht werden kann.

Es ist bereits bekannt, daß L-PTC durch saure Hydrolyse (JA-OS 73-85538) oder durch enzymatischen Abbau (JA-OS 74-31890) von L-PTC-alanyl-alanin, einem literaturbekannten, mikrobiell gewonnenen Antibiotikum, erhalten werden kann.

Außerdem sind Verfahren bekannt, die auf der enzymatischen Racematspaltung chemisch synthetisierter racemischer PTC-Vorstufen basieren. In der DE-A 2939269 wird ein Verfahren beschrieben, bei dem N-Acyl-PTC, insbesondere N-Acetyl-PTC, mit Hilfe von Acylasen gespalten wird, die aus speziell gezüchteten Mikrobenstämmen der Gattung Pseudomonas, Streptomyces oder Aspergillus erhältlich sind.

In der EP 0 382 113 sind verschiedene Verfahren beschrieben, bei denen racemische 2-Acylamino-4-(alkoxy-methylphosphinyl) butancarbonsäureester mittels Esterasen bzw. 2-Acylamino-4-(alkoxy-methylphosphinyl)-butansäureester- und amide mittels Acylasen enzymatisch gespalten werden.

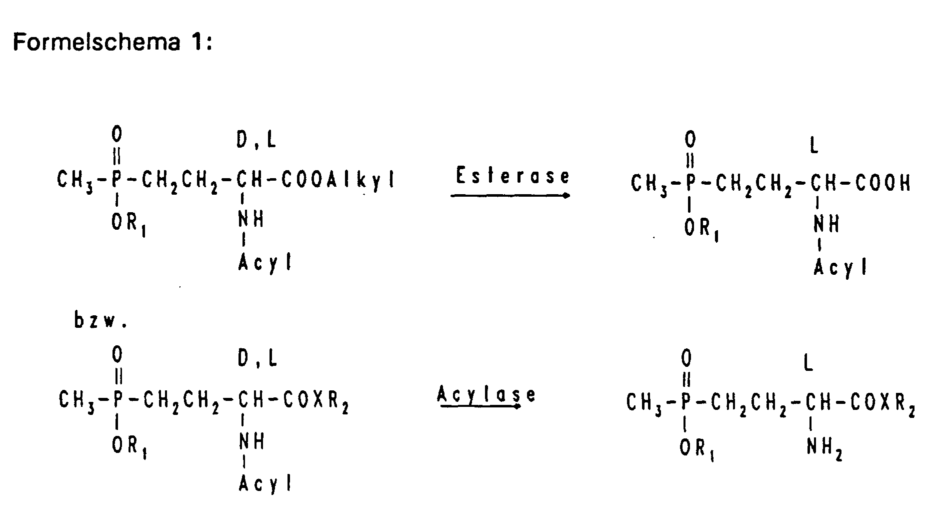

In WO 8910969 A wird ein Verfahren beschrieben, bei dem insbesondere arylsubstituierte racemische Aminonitrile zunächst durch Inkubation mit einer Kultur von Acinetobacter calcoatiens (DSM 3875) in die entsprechenden D,L-Aminosäureamide überführt werden, die in einem weiteren Schritt durch eine L-Aminosäureamidase, wie z.B. Arthrobacter ATCC 31652, zu L-Aminosäure gespalten werden, wobei gleichzeitig das zurückbleibende D-Aminosäureamid durch eine Amidracemase racemisiert wird (Formelschema 2). 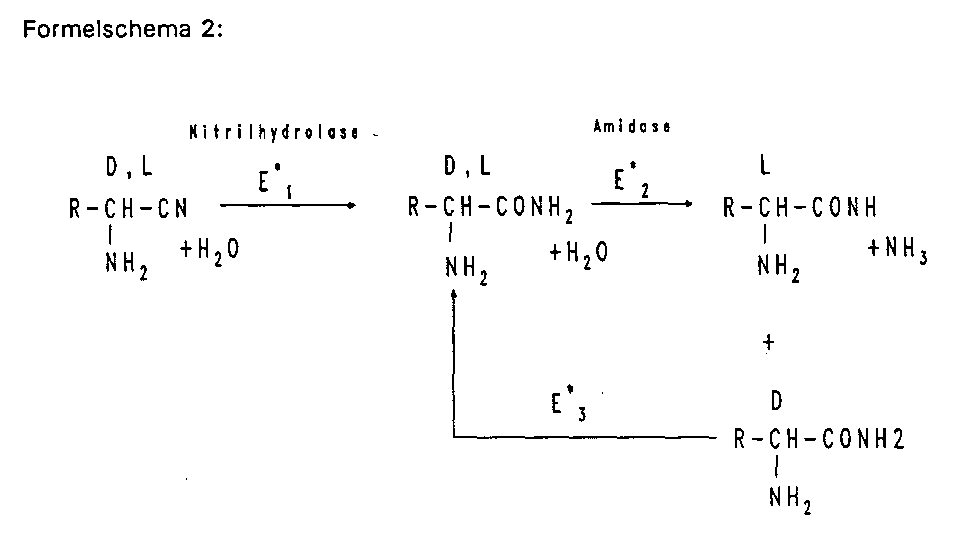
R = z.B. -CH₂C₆H₅;
E₁^{*} = Aminonitrilhydrolase
E₂^{*} = L-Aminosäureamidamidase
E₃^{*} = D-Aminosäureamidracemase

Auch wenn diese verschiedenen Syntheseschritte nacheinander oder simultan durchgeführt werden können, erscheint das Verfahren wegen der Komplexizität der Teilverfahren mit drei verschiedenen Enzymen für die Realisierung technischer Produktionen von L-Aminosäuren als wenig aussichtsreich.

Es wird nicht auf eine Möglichkeit der Spaltung von Aminosäuren mit phosphorhaltigen Resten hingewiesen. Dies ist wohl darauf zurückzuführen, daß P(V)-haltige Verbindungen, insbesondere Derivate der allgemeinen Formel R'R"P(O)OR"' aufgrund ihrer sterischen Anordnung den Übergangszustand eines enzymatisch hydrolysierten Carboxylesters simulieren (siehe M. Dixon, E. Webb in "Enzymes" e. Edition, Longmans, Gren & Co LTD, London 1964, S. 346-352) und demzufolge einen desaktivierenden Einfluß ausüben können.

In J. Org. Chem. 53, 1826 (1988) wird weiterhin ein Verfahren zur enzymatischen Trennung von racemischen α-alkylierten Aminosäureamiden mit Mycobacterium nesaurum (ATCC 25725) zu α-alkylierten L-Aminosäuren und den entsprechenden D-Aminosäureamiden beschrieben, wobei die erzielten enantiomeren Überschüsse nur mäßige Werte erreichen.

In der EP-A 193 113 werden racemische α-Aminosäureamide wie z.B. D,L-Phenylalaninamid durch Microorganismen wie z.B. Pseudomonas zu L-Aminosäuren hydrolisiert. Das verbleibende D-Aminosäureamid wird durch starke Basen wie NaOH racemisiert und in den Prozeß zurückgeführt. Auch hier fehlt in der Patentbeschreibung jeder Hinweis auf die Verwendung phosphorhaltiger Aminosäurederivate.

Schließlich wird in Bull. Chem. Soc. Japan 61, 3599 (1988) die Hydrolyse von L-2-Acetamido-4-(methylphosphinyl)-butanamid bzw. von L-2-Acetamido-4-(ethoxy-methylphosphinyl)-butanamid zu L-2-Acetamido-4-(methylphosphinyl)-butansäure bzw. zu L-2-Acetamido-4-(ethoxy-methylphosphinyl)-butansäure durch das Enzym Glutaminase beschrieben. Diese Amidasereaktionen werden jedoch ausdrücklich nur mit den entsprechenden L-konfigurierten Phosphinothricinamidderivaten durchgeführt, sie zeigen somit keinen Weg zur ökonomischen Synthese von L-Phosphinothricin aus billigen racemischen Vorstufen auf.

Aufgrund des genannten Standes der Technik war es nicht vorherzusehen, daß mit einfach zugänglichen Phosphinothricinamiden, die am Phosphinsäureteil modifiziert sind, eine effektive enzymatische Synthese von L-Phosphinothricin durchgeführt werden konnte.

Gegenstand der Erfindung ist ein Verfahren zur enzymatischen Trennung von Derivaten, das dadurch gekennzeichnet ist, daß man ein Gemisch von D-und L-Derivaten der allgemeinen Formeln (I) und (II) 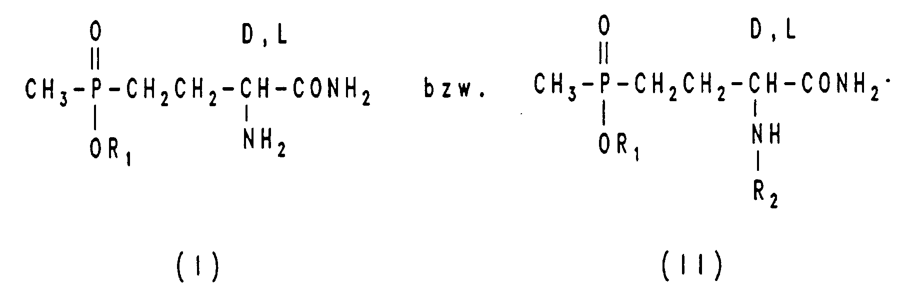 in welcher
- R¹: für unverzweigtes oder verzweigtes (C₁-C₂₀)-Alkyl, das unsubstituiert oder durch ein oder mehrere Halogenreste, wie Fluor, Chlor, Brom oder Jod, oder ein- oder mehrfach durch (C₁-C₈)-Alkoxy substituiert ist, oder für (C₃-C₈)-Cycloalkyl, das durch eine oder mehrere Gruppen (C₁-C₄)-Alkyl, (C₁-C₄)-Alkoxy und Halogen substituiert sein kann, oder für (C₃-C₁₀)-Alkenyl, (C₃-C₁₀)-Alkinyl oder Benzyl steht, und
- R²: für Formyl, unverzweigtes oder verweigtes (C₁-C₂₀)-Alkyl-carbonyl, das unsubstituiert oder im Alkylteil durch einen oder mehrere Reste aus Hydroxy, Halogen, Phenyl, welches durch bis zu 3 Reste aus der Gruppe (C₁-C₁₂)-Alkyl, (C₁-C₁₂)-Alkoxy, Halogen, Nitro und CF₃ substituiert sein kann, (C₁-C₄)-Alkoxy und (C₁-C₄)-Alkylthio substituiert ist, für Benzoyl oder Benzoyl, das durch 1 bis 3 Reste aus der Gruppe (C₁-C₁₂)-Alkyl, (C₁-C₁₂)-Alkoxy, Halogen, Nitro und CF₃ substituiert ist, steht,
im wässrigen oder wässrig-organischen Medium mit einem hydrolytisch aktiven Enzym behandelt, wobei ein selektiv wirkendes L-Aminosäureamidspaltendes Enzym bzw. Mikroorganismus verwendet wird.

Hierbei können racemische oder auch in der L-Form angereicherte Aminosäureamidderivate der allgemeinen Formeln (I) bzw. (II) eingesetzt werden. Das verbleibende D-Phosphinothricin-amidderivat kann z.B. durch Einwirkung von Basen racemisiert und in den Prozeß zurückgeführt werden.

Auch kann die Reaktion in Gegenwart einer D-Aminosäureamidracemase durchgeführt werden, so daß schließlich reine L-Phosphinothricinderivate erhalten werden.

Von besonderem Interesse ist ein erfindungsgemäßes Verfahren, dadurch gekennzeichnet, daß man ein Gemisch von D- und L-PTC-Derivaten der genannten Formeln (I) bzw. (II) verwendet, in welchen
- R¹: für unverzweigtes oder verzweigtes (C₁-C₁₀)-Alkyl oder (C₁-C₁₀)-Alkyl, das durch Halogen, wie Fluor, Chlor oder durch (C₁-C₄)-Alkoxy substituiert ist, oder für (C₅-C₆)-Cycloalkyl steht, und
- R²: für Wasserstoff, Formyl, unverzweigtes oder verweigtes (C₁-C₁₀)-Alkyl-carbonyl, das unsubstituiert oder im Alkylteil durch einen oder zwei Reste aus der Gruppe Hydroxy, Halogen, Phenyl oder Phenyl, welches durch 1 bis 3 Reste aus (C₁-C₄)-Alkyl, (C₁-C₄)-Alkoxy und Halogen substituiert ist, (C₁-C₄)-Alkoxy und (C₁-C₄)-Alkythio substituiert ist, oder für Benzoyl oder Benzoyl, das durch 1 bis 3 Reste aus der Gruppe C₁-C₄-Alkyl, C₁-C₄-Alkoxy und Halogen substituiert ist, steht.

Bevorzugt ist ein erfindungsgemäßes Verfahren, dadurch gekennzeichnet, daß man ein Gemisch von D- und L-PTC-Derivaten der genannten Formeln (I) bzw. (II) verwendet, in welchen
- R¹: für unverzweigtes oder verzweigtes (C₁-C₁₀)-Alkyl steht,
- R²: für Wasserstoff, (C₁-C₁₀)-Alkyl-carbonyl, das durch Phenyl oder durch ein- bis dreifach substituiertes Phenyl, dessen 1 bis 3 Substituenten aus (C₁-C₄)-Alkyl, (C₁-C₄)-Alkoxy und Halogen ausgewählt sind, substituiert ist, vorzugsweise Phenacetyl, oder für Benzoyl steht.

(C₁-C₆)-Alkyl bedeutet insbesondere Methyl, Ethyl, 1-Propyl oder 2-Propyl, n-, i-, tert- oder 2-Butyl, 3-Methyl-but-2-yl, n-, i-, tert-, 2- oder 3-Pentyl, n-hexyl oder ein stereoisomeres Hexyl. C₁-C₆-Alkoxy bedeutet insbesondere (C₁-C₆-Alkyl)-oxy, wobei der Alkylrest dabei die vorstehend genannte Bedeutung hat.

Wenn nicht näher definiert bedeutet Halogen die Reste Fluor, Chlor, Brom und Jod, vorzugsweise Fluor und Chlor, insbesondere Chlor.

Die erfindungsgemäße Spaltung läßt sich verwirklichen durch N-Acylspaltung der D,L-PTC-Derivate der Formeln (I) bzw. (II), in denen R¹ und R² die oben genannten Bedeutungen haben.

Bei dieser Amidasespaltung der Aminosäureamidderivate der allgemeinen Formel (I) fällt das entsprechende phosphinestergeschützte L-Phosphinothricin der allgemeinen Formel (III) an, 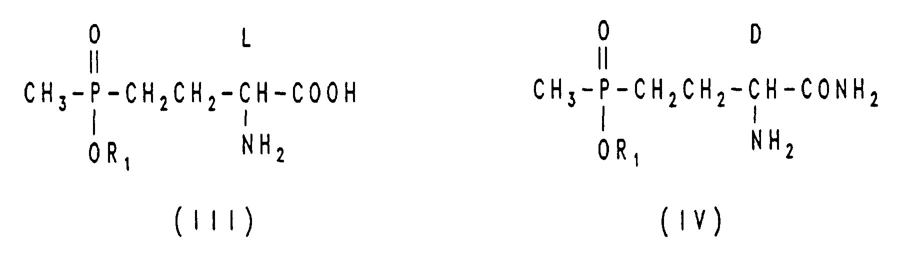 das sich auf bekanntem Wege von dem nicht umgesetzten Amid der Formel IV in an sich üblicher Weise isolieren läßt, z.B. durch extraktive Abtrennung des nicht umgesetzten D-PTC-Derivats von der abgespaltenen Säure bei einem pH-Wert im sauren Bereich, wobei das L-PTC-Derivat in der wässrigen Lösung als Ammoniumsalz verbleibt und anschließend durch Einengen der wässrigen Lösung zur Trockne isoliert werden kann. Weiterhin ist eine Trennung durch Kristallisation, Destillation, Chromatographie u.a. denkbar.

Bei der Amidasespaltung dieser N-Acyl-Aminosäurederivate der allgemeinen Formel (II) fällt das entsprechende phosphinestergeschützte L-N-Acylphosphinothricin der allgemeinen Formel V im Gemisch mit nicht umgesetzten D-N-Acylaminosäureamid der allgemeinen Formel VI an.

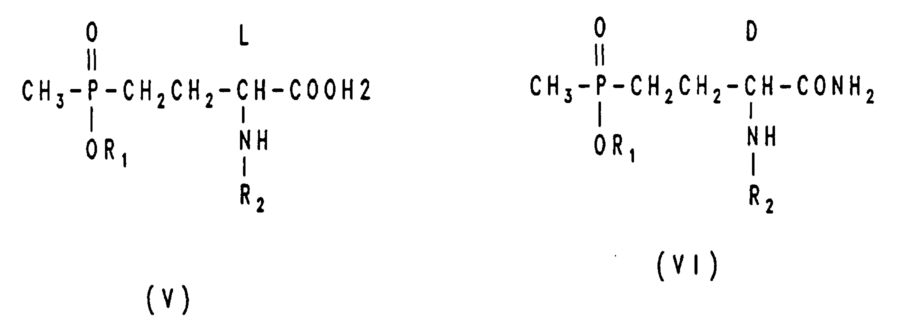

Die Isolierung des enzymatisch hydrolisierten L-PTC-Derivats V gelingt nach im Prinzip bekannter oder üblicher Weise, wie z.B. durch Kristallisation, Destillation, Säulen- oder lonenaustauscherchromatographie und insbesondere durch schnelle Extraktion, gegebenenfalls bei reduzierter Temperatur von 0 bis 10°C, des nicht umgesetzten D-PTC-Derivats aus der wässrigen Phase (der pH-Wert liegt vorzugsweise zwischen 9 und 11, insbesondere zwischen 9,5 und 10,5) mit Hilfe eines geeigneten organischen Lösungsmittels, wobei das aus der Amidspaltung entstandene L-PTC-Derivat als Carboxylat-Salz in der wässrigen Phase verbleibt.

Nach Abtrennung des D-Derivats erfolgt die chemische Hydrolyse des L-PTC-Derivats analog üblichen Methoden, beispielsweise mit wässriger verdünnter Mineralsäure bei einer Temperatur von 20°C bis Siedetemperatur der Lösung bei Reaktionszeiten von 1 bis 24 Std. Als Mineralsäure eignet sich beispielsweise eine Halogenwasserstoffsäure oder Schwefelsäure, insbesondere verdünnte bis konzentrierte Salzsäure.

Aber auch organische Säuren sind in einzelnen Fällen gut geeignet. Bei der chemischen Totalhydrolyse fällt neben L-PTC auch die dem Acylrest R² entsprechende Carbonsäure an, wobei letztere durch Destillation oder Extraktion mit einem geeigneten organischen Lösungsmittel aus der wässrig-sauren Lösung entfernt werden kann.

Die verbleibenden D-PTC-Derivate lassen sich gegebenenfalls durch Extraktionsverfahren, Destillation, Kristallisation oder Chromatographie von Nebenprodukten der enzymatischen Hydrolyse reinigen und eventuell nach Racemisierung, z.B. auf thermische Weise oder durch Einwirkung einer Base, wie eines Alkoholats in alkoholischer Lösung, als D,L-Gemisch erneut der enzymatischen Hydrolyse unterwerfen. Diese Racemisierung mit Base verläuft in der Regel besonders substanzschonend.

Die besagten Enzyme können in freier Form oder nach immobilisierung gemäß dem Fachmann bekannten oder üblichen Verfahren eingesetzt werden. Das jeweilige Substrat wird im wässrigen Medium gelöst bzw. suspendiert eingesetzt. Konzentrationen von 0,1 % bis zur gesättigten Lösung (bei Arbeiten in Suspension) sind möglich. Der Zusatz von wasserlöslichen Lösungsmitteln wie Methanol ist im Einzelfall ebenso praktizierbar wie das Arbeiten im Zweiphasengebiet unter Zusatz wasserunlöslicher organischer Lösungsmittel.

Die Reaktionstemperatur für die enzymatischen Spaltungen ist in der Regel 10-60°C, bevorzugt 20 - 40°C. Das Verfahren kann beispielsweise als Batchverfahren oder kontinuierlich als Säulenverfahren durchgeführt werden.

Die enzymatische Hydrolyse wird vorzugsweise bei einem pH von 5 bis 12, insbesondere pH 6 bis 10 durchgeführt, wobei im Einzelfall zwecks Unterdrückung einer unspezifischen Hydrolyse des Carboxylesters -bzw. - amids auch bei pH 5 bis 7 gearbeitet werden kann.

Der Verlauf der Reaktionen kann beispielsweise über HPLC anhand der Abnahme des Substrats verfolgt werden. Im Einzelfall, insbesondere bei der Carbonsäureesterspaltung, läßt sich der Reaktionsverlauf auch durch andere einfache Methoden, z.B. durch die Menge an erforderlicher zudosierter Base bis zur pH-Konstanz, ermitteln.

Der Gehalt an L-Verbindung im Produkt der enzymatischen Spaltung kann nach erfolgter alkalischer oder saurer Hydrolyse zum L-PTC und anschließender Derivatisierung nach im Prinzip bekannter Weise (D. Asward, Analytical Biochemistry 137, 405-409 (1984) mit Hilfe von HPLC bestimmt werden.

Die Ausgangsstoffe der allgemeinen Formel II sind bekannt oder lassen sich nach an sich bekannten Verfahren herstellen (vgl EP 0 382 114).

Die Ausgangsstoffe der allgemeine Formel I sind bisher unbekannt und daher ebenfalls Gegenstand der vorliegenden Patentanmeldung. Die Darstellung dieser Verbindungen ist exemplarisch im experimentellen Teil aufgeführt.

Die Bezeichnung D bzw. L bei den gegebenenfalls derivatisierten PTC-Derivaten gibt nur die Konfiguration an dem in α-Stellung zur Carbonsäuregruppe befindlichen C-Atom an, das mit der gegebenenfalls geschützten Aminogruppe verbunden ist. Wegen des zusätzlichen Chiralitätszentrums am Phosphoratom in den mit R¹ geschützten PTC-Derivaten sind diese D- bzw. L-PTC-Derivate in der Regel keine reinen Enantiomeren, sondern Gemische von Diastereomeren.

Für die enzymatische Spaltung ist, wie sich überraschenderweise gezeigt hat, im wesentlichen nur die Konfiguration am erwähnten α-C-Atom von Bedeutung. Nach Hydrolyse des Restes R¹ geht das Chiralitätszentrum am Phosphoratom wegen des schnellen Protonenaustauschs zwischen OH und O praktisch verloren.

Das erfindungsgemäße Verfahren zur Trennung von D,L-PTC-Derivaten und zur Herstellung von L-PTC zeichnet sich durch geringen Salzanfall bei effektiver Trennung von Substrat- und Produktgemisch aus. Das nach der enzymatischen Trennung anfallende D-PTC-Derivat kann, falls nicht erwünscht, leicht und schonend, gegebenenfalls ohne vorherige Isolierung, racemisiert und somit für eine erneute enzymatische Trennung verwendet werden.

Das erfindungsgemäße Verfahren kann vorzugsweise mit Mikroorganismen durchgeführt werden, die Amidasen synthetisieren. Solche Mikroorganismen sind z. B. Enterobacter aerogenes (DSM 9164), Klebsiella oxytoca (DSM 9162) Klebsiella trevisanii (DSM 9163), Corynebacterium aquaticum (DSM 9171), Rhodococcus rubropertinctus (ATCC 21930), Rhodococcus rhodochrous (ATCC 33278), Arthrobacter sp.(ATCC 31652) und Corynebacterium sp. (ATCC 31662).

Ein besonderer und unerwarteter Vorteil besteht in der Möglichkeit, bei Einsatz der PTC-Derivate mit erfindungsgemäß estergeschützter P-O-Säurefunktion sogar kommerziell erhältliche Amidasen zur enantioselektiven Spaltung der PTC-Derivate verwenden zu können. Eine Hemmung der hydrolytischen Aktivität durch den Phosphinsäureester oder gar die Nebenreaktion, eine Hydrolyse des Phosphinsäureesters, wird dabei überraschenderweise in der Regel nicht beobachtet. Bei dieser enzymatischen Amidspaltung wirkt sich der Schutz der Phosphinsäure als Ester positiv auf die Umsatzgeschwindigkeit aus bzw. macht im Einzelfall die Akzeptanz des Substrats durch das Enzym erst möglich.

### A) Ausgangsmaterialien

### Beispiel A 1

### D,L-2-Amino-4-(1-butoxy-(methyl)phosphinyl)butansäureamid (Formel I: R₁ =n-C₄H₉)

### a) D,L-2-Amino-4-(1-butoxy-(methyl)phosphinoyl)butansäurenitril (Herstellung analog U.S. 5,051,525 oder EP 0 382 114).

60 ml Ammoniaklösung (25%ig) werden bei 30°C innerhalb von 2 Stunden mit 52,25 g (0,200 mol) D,L-2-Acetoxy-4-(1-butoxy-(methyl)phosphinoyl)butyronitril versetzt.

Anschließend wird das Reaktionsgemisch mit Dichlormethan extrahiert, der Extrakt über Natriumsulfat getrocknet und einrotiert. Man erhält 32,1g (73,5% d.Th.) eines gelblichen Öls, das ohne Reinigung in der nächsten Synthesestufe eingesetzt wird.

### b) D,L-2-Amino-4-(1-butoxy-(methyl)phosphinoyl)butansäureamid

16,14 g D,L-2-Amino-4-(1-butoxy-(methyl)phosphinoyl)butansäurenitril (0,075 mol) werden in 100 ml Ameisensäure bei Raumtemperatur mit 60 g HCL-Gas gesättigt. Nach 2 Stunden wird das Reaktionsgemisch eingeengt, in Wasser aufgenommen, mit gesättigter Natriumbicarbonatlösung auf einen pH-Wert von 8 eingestellt. Die Lösung wird anschließend zweimal mit Dichlormethan gewaschen, anschließend wird die wässrige Phase einrotiert und mehrfach mit Methanol eingekocht. Nach Einengen der Methanolextrakte wird das zurückbleibende Öl in Methanol/Dichlormethan (3:7) an Kieselgel chromatographiert.

Man erhält 14,7 g (83,1% d.Th) an D,L-2-Amino-4-(1-butoxymethyl)phosphinoyl)butansäureamid in Form eines farblosen Öls.
¹H-NMR (CDCl₃): 0.94 (t, J=Hz, 3H); 1.48 (d, J=14Hz, 3H), overlapped by 1.32-1.48 (m, 2H), 1.64 (m, 2H), 1.8-2.1 (n, 4H); 2.5 (s, NH₂); 3.52 (t, J= ..Hz), 3.96 (m, 2H); 6.38 (s, 1H); 7.42 (g, 1H). ²¹p-NMR(D₂..): 62.4 (s) ppm.

### Biologische Beispiele

### Substanzen:

D,L-2-Amino-4-(methyl)(n-butoxy)phosphino-yl-butansäureamid = PPT-AM D,L-N-Acetyl-4-(methyl)(n-butoxy)phosphino-yl-2-aminobutansäureamid = PPT-Ac

### Beispiel 1: Abbau von PPT-AM und PPT-Ac im Boden:

Je 10 g Boden verschiedener Herkunft: (sandig, Uferkies, Waldhumus, Wiese, Gartenhumus, Lehm) wurde getrocknet, gesiebt und mit je 1,2 ml von 100 mM Lösungen der beiden Testsubstanzen, pH = 7,0 versetzt. Die Ansätze wurden für 14 Tage bei Raumtemperatur im Dunkeln inkubiert.

Zur Analyse des Abbauverhaltens wurden nach 1, 2, 4, 6, 8, 12 und 14 Tagen Proben von 0,5 g abgenommen und mit 2 x 0,5 ml Wasser 2 x 30 min. bei 60°C extrahiert. Die vereinigten Überstände wurden im Aminosäureanalysator mit Ninhydrin- Nachsäulenderivatisierung (Biotronic LC5001) (für PPT-AM) bzw. durch HPLC (Säule: Aminex HPX-87H/Eluent: 0,01 H₂SO₄, 10% Acetonitril bzw. 0,1 % Trifluoracetat, 10 % Acetonitrill/Elution: isokratisch Detektion: 195 nm bzw. 210 nm) (für PPT-Ac) untersucht.

Kontrollversuche wurden mit sterilem Boden (4 h bei 200°C inkubiert) durchgeführt. Es zeigte sich, daß beide Testsubstanzen in den mikrobiell aktiven Böden mit einer Halbwertzeit von ca. 1-2 Tagen abgebaut werden. Dabei akkumuliert jeweils ein Metabolit in etwa äquimolarem Verhältnis zur Ausgangssubstanz. Ein weitergehender Abbau der Testverbindungen konnte innerhalb des Versuchszeitraumes von 2 Wochen nicht festgestellt werden. In den sterilen Böden war keine Umsetzung der beiden Amide nachweisbar.

### Beispiel 2: Anreicherungskulturen von Bodenmikroorganismen mit PPT-AM bzw. PPT-Ac als alleiniger Stickstoff-Quelle:

Je 1 g der in Beispiel 1 beschriebenen Böden wurde für 1 h bei Raumtemperatur mit 10 mM NaCI, 10 mM NaPhosphat-Puffer, pH=7,0 extrahiert und die Überstände in folgendes Medium angeimpft:
0,2 % Glucose
5 mM Succinat
10mM Glycerin
1 g/l NH₄Cl
2,5 g/l K₂HPO₄, pH=7,2
0,06 g/l MgSO₄
0,01 g/l NaCI
1 ml/l Spurenelemente-Lösung
10 mM PPT-AM bzw. PPT-Ac

| | |
|---|---|
| Spurenelemente-Lösung | 1 g/l FeSO₄ x 7 H₂O |
| | 0,22 g/l MnSO₄ x H₂O |
| | 0,1 g/l H₃BO₃ |
| | 0,1 g/l Na₂MoO₄ x 2 H₂O |
| | 0,18 g/l ZnSO₄ x 7 H₂O |
| | 0,16 g/l CuSO₄ x 5 H₂O |
| | 0,1 g/l CoCl₂ x 6 H₂O |
| | 1 ml/l 1N HCl. |

Je 2 ml dieser Kulturen wurden für 2-3 Tage bei 28°C und 200 rpm inkubiert und nach erfolgtem Wachstum unter schrittweiser Reduktion der Stickstoffquelle NH₄Cl (1 g/l --> 0,5 -->0,1 g/l --> 0 g/l) weiterpassagiert. Auf diesem Wege konnten mehrere Anreicherungskulturen gewonnen werden, die mit PPT-AM bzw. PPT-Ac als alleiniger Stickstoffquelle wachsen können.

Zur Gewinnung von Reinkulturen wurden aus den Anreicherungskulturen nach Ausplattierung auf dem entsprechenden Agar-Medium Einzelkolonien isoliert, diese wieder in Flüssigmedium vermehrt und zur Überprüfung der Reinheit erneut auf Agar-Platten ausgestrichen. Die erhaltenen Reinkulturen konnten wie folgt identifiziert werden:
- Nutzung von PPT-AM und PPT-Ac als alleinige Stickstoff-Quellen: Enterobacter aerogenes (DSM-Hinterlegungsnummer: DSM 9164)
- Nutzung von PPT-Ac als alleinige Stickstoff-Quelle:
   Klebsiella oxytoca (DSM-Hinterlegungsnummer: DSM 9162)
   Klebsiella trevisanii (DSM-Hinterlegungsnummer: DSM 9163)
   Corynebacterium aquaticum (DSM-Hinterlegungsnummer: DSM 9171)

### Beispiel 3: Screening weiterer Mikroorganismen auf Verwertung von PPT-AM bzw. PPT-Ac als alleinige Stickstoff-Quelle:

Nach dem in Beispiel 2 beschriebenen Schema wurden eine Reihe von Mikroorganismen aus Stammsammlungen auf Verwertung der beiden Amide als einzige Stickstoff-Quelle gescreent. Dabei wurden folgende Stämme gefunden, die ausschließlich PPT-AM als Substrat verwerten können:
Rhodococcus rubropertinctus, ATCC 21930
Rhodococcus rhodochrous, ATCC 33278
Arthrobacter sp., ATCC 31652
Corynebacterium sp., ATCC 31662

### Beispiel 4: Charakterisierung der Abbaureaktionen:

Die in den Beispielen 2 und 3 beschriebenen Stämme wurden in 10 ml Kulturen in Minimalmedium gemäß Beispiel 2 mit dem jeweiligen Amidsubstrat als einziger Stickstoffquelle für 2-3 Tage bei 28°C und 200 rpm angezogen. Die Zellen wurden in der spätlogarithmischen Wachstumsphase abzentrifugiert und die Kulturuberstände im Aminosäureanalysator (für PPT-AM-Kulturen) bzw. in der HPLC (für PPT-Ac-Kulturen), wie in Beispiel 1 dargestellt, analysiert.

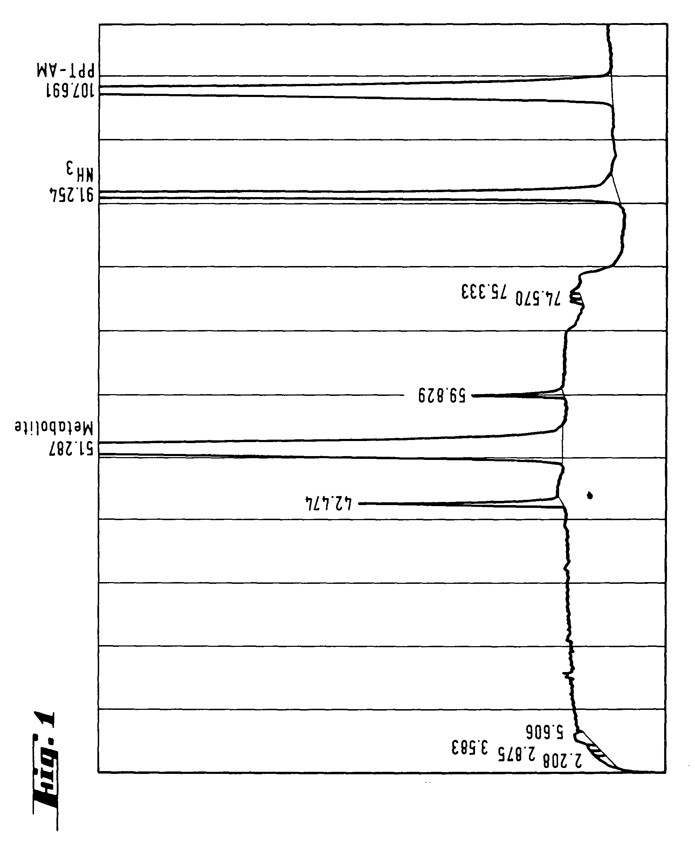

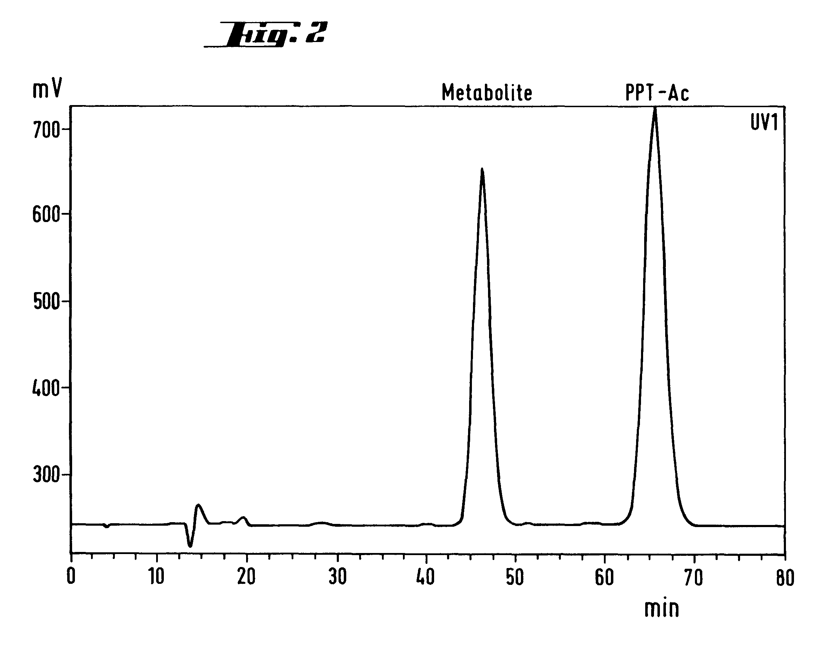

Bei beiden Amid-Substraten konnte die Bildung eines Metaboliten beobachtet werden, der zur nicht umgesetzten Ausgangsverbindung in etwa äquimolarem Verhältnis steht (siehe Abb. 1 und 2).

Eine weitergehende Umsetzung der racemischen Amide PPT-AM und PPT-Ac konnte trotz des Überschusses an Kohlenstoff-Quellen im Medium nicht beobachtet werden, was auf eine Stereoselektivität der Abbaureaktionen hinweist. Außerdem konnte in den Kulturüberständen eine Anreicherung von Ammoniak nachgewiesen werden (siehe Abb. 1), das bei Amidase-Reaktionen als Produkt entsteht.

### Beispiel 5: Identifizierung des PPT-Ac Metabolite und Bestimmung der Enantiomerenverhältnisse:

Die Kulturüberstände aus Beispiel 4 (PPT-Ac) wurden durch Gefriertrocknung 10 fach konzentriert. Aus diesen Lösungen wurden je ca. 10 mg der Metabolite sowie der nicht umgesetzten Ausgangsverbindungen wie folgt isoliert:
- PPT-Ac und Metabolit M: präparative HPLC, Bedingungen siehe Beispiel 1,
   Elution: 0,1 % Trifluoracetat, 10 % Acetonitril,
   Detektion: 210 nm.

Die isolierten Substanzen wurden durch Gefriertrocknung eingeengt und anschließend massenspektroskopisch analysiert. Damit konnten die Molekülstrukturen des Metaboliten ermittelt werden. Es handelt sich um:
- M = N-Acetyl-4-(methyl)(n-butoxy)phosphino-yl-2-aminobutansäure.

Zur Untersuchung der Enantiomerenreinheit wurde der Metabolit sowie die nicht umgesetzten Amide mit HCI zum freien Phosphinothricin (PPT) hydrolysiert. Die Enantiomerenverhältnisse der so erhaltenen PPT-Proben wurden nach Derivatisierung mit Hilfe der chiralen HPLC (D. Aswad, Analytical Biochemistry, 137, 405-409, 1984) bestimmt.

Die Ergebnisse sind hier für den Stamm Enterobacter aerogenes (DSM - Hinterlegungsnummer: DSM 9164) dargestellt:

| | | | |
|---|---|---|---|
| - Metabolit | 95,7 % L-PPT | - nicht umgesetztes PPT-AM | 3,3 % L-PPT |
| | 4,3 % D-PPT | | 96,7 % D-PPT |

Vergleichbare Resultate wurden auch für die anderen hier beschriebenen Stämme erzielt.

Aus den Untersuchungen geht hervor, daß es sich bei dem gefundenen Reaktionstyp um eine stereoselektive Hydrolyse der eingesetzten racemischen Amide zu den entsprechenden L-Carbonsäuren handelt, die durch L-spezifische Amidasen katalysiert werden.

### Beispiel 6: Enantioselektive Amidspaltung von PPT-Ac durch Biotransformation:

Klebsiella oxytoca (DSM-Hinterlegungsnummer: DSM 9162) wurde in 100 ml Medium gemäß Beispiel 2 mit 10 mM PPT-Ac als alleiniger Stickstoff-Quelle für 2 Tage bei 28°C und 200 rpm im Erlenmeyerkolben kultiviert. Anschließend wurden die Zellen für 10 min. bei 500 rpm abzentrifugiert, 1 x in 50 ml 10 mM NaCl, 10 mM NaPhosphat-Puffer, pH = 7,0 gewaschen und mit einer Konzentration von c = 50 mg/ml im gleichen Puffer mit 30 mg/ml D,L-PPT-Ac resuspendiert.

Der Biotransformationsansatz (12 ml Gesamtvolumen) wurde bei 28°C und 200 rpm für 15 h im Erlenmeyerkolben inkubiert und der Reaktions- überstand danach in der HPLC (siehe Beispiel 1) gemessen.

Der Gehalt an N-Acetyl-4-(methyl)(n-butoxy)phosphino-yl-2-aminobutansäure betrug 14,5 mg/ml. Die Enantiomerenreinheit des gebildeten Produktes entsprach dem in Beispiel 5 angegebenen Wert.
- Abb. 1:: Aminosäureprofil des Kulturüberstandes von Enterobacter aerogenes (DSM-Hinterlegungsnummer: DSM 9164), angezogen in Minimalmedium mit 10 mM PPT-AM als alleiniger Stickstoff-Quelle (siehe Beispiel 2). Die Probe wurde im Aminosäureanalysator, Modell Biotronic LC5001, mit Ninhydrin-Nachsäulenderivatisierung gemessen. Die Zahlen geben die Retentionszeiten derjeweiligen Peaks in Minuten an.
51.287 min. = 2-Amino-4-(methyl)(n-butoxy)phosphino-yl-butansäure
91.254 min. = Ammoniak
107.691 min. = PPT-AM.
   Die anderen Peaks sind interne Peaks des Meßgerätes.
- Abb.2:: HPLC-Profil des Kulturuberstandes von Enterobacter aerogenes (DSM-Hinterlegungsnummer: DSM 9164), angezogen in Minimalmedium mit 10 mM PPT-Ac als alleiniger Stickstoff-Quelle (siehe Beispiel 2). Die Probe wurde mit der Säule Aminex HPX-87H gemessen, isokratische Elution mit 0,1 % Trifluoracetat, 10 % Acetonitril, Detektion: 210 nm.
M2 = N-Acetyl-4-(methyl)(n-butoxy)phosphino-yl-2-aminobutansäure.

## Patentansprüche

1. Verfahren zur enzymatischen Trennung von PTC-Derivaten, **dadurch gekennzeichnet, daß** man ein Gemisch von D- und L-PTC-Derivaten der allgemeinen Formeln (I) und (II) 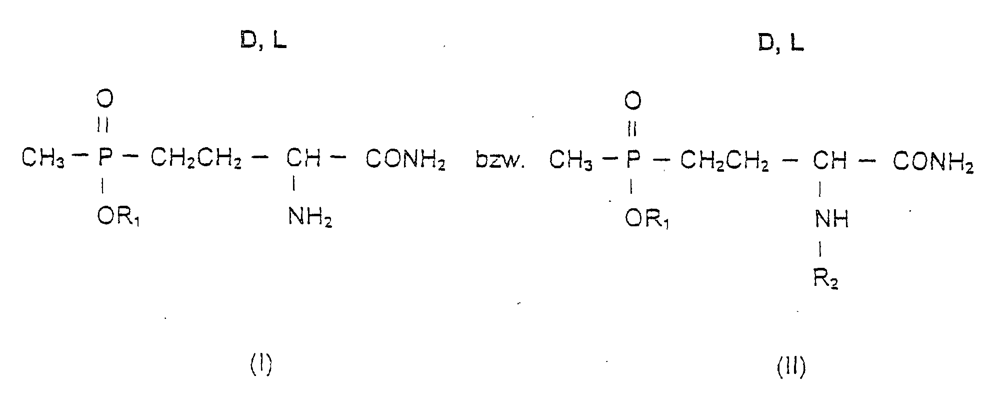 in welcher
R¹ für unverzweigtes oder verzweigtes (C₁-C₂₀)-Alkyl, das unsubstituiert oder durch ein oder mehrere Halogenreste, wie Fluor, Chlor, Brom oder Jod, oder ein- oder mehrfach durch (C₁-C₈)-Alkoxy substituiert ist, oder für (C₃-C₈)-Cycloalkyl, das durch eine oder mehrere Gruppen (C₁-C₄)-Alkyl, (C₁-C₄)-Alkoxy und Halogen substituiert sein kann, oder für (C₃-C₁₀)-Alkenyl, (C₃-C₁₀)-Alkinyl oder Benzyl steht, und
R² für Formyl, unverzweigtes oder verzweigtes (C₁-C₂₀)-Alkyl-carbonyl, das unsubstituiert oder im Alkylteil durch einen oder mehrere Reste aus Hydroxy, Halogen, Phenyl, welches durch bis zu 3 Reste aus der Gruppe (C₁-C₁₂)-Alkyl, (C₁-C₁₂)-Alkoxy Halogen, Nitro und CF₃ substituiert sein kann, (C₁-C₄)-Alkoxy und (C₁-C₄)-Alkylthio substituiert ist, für Benzoyl oder Benzoyl, das durch 1 bis 3 Reste aus der Gruppe (C₁-C₁₂)-Alkyl, (C₁-C₁₂)-Alkoxy, Halogen, Nitro und CF₃ substituiert ist, steht,
im wässrigen oder wässrig-organischen Medium mit einem hydrolytisch aktiven Enzym behandelt, wobei ein selektiv wirkendes L-Aminosäureamidspaltendes Enzym bzw. Mikroorganismus verwendet wird.

2. Verfahren gemäß Anspruch 1, in dem ein selektiv wirkendes L-Aminosäure-amidspaltendes Enzym oder ein Mikroorganismus, der dieses Enzym synthetisiert, verwendet wird.

3. Verfahren gemäß Anspruch 1, in dem mindestens ein Mikroorganismus aus der Gruppe, welche aus Enterobacter aerogenes (DSM 9164), Klebsiella oxytoca (DSM 9162) Klebsiella trevisanii (DSM 9163), Corynebacterium aquaticum (DSM 9171), Rhodococcus rubropertinctus (ATCC 21930), Rhodococcus rhodochrous (ATCC 33278), Arthrobacter sp.(ATCC 31652) und Corynebacterium sp. (ATCC 31662) besteht, verwendet wird.

4. Verfahren gemäß Anspruch 1, in dem racemische oder in der L-Form angereicherte Aminosäureamidderivate der allgemeinen Formeln (I) bzw. (II) eingesetzt werden.

5. Verfahren gemäß Anspruch 1, in dem die Reaktion in Gegenwart einer D-Aminosäureamidracemase durchgeführt wird.

6. Verfahren gemäß Anspruch 1, in welchem
R¹ für unversweigtes oder verzweigtes (C₁-C₁₀)-Alkyl oder (C₁-C₁₀)-Alkyl, das durch Halogen, wie Fluor, Chlor oder durch (C₁-C₄)-Alkoxy substituiert ist, oder für (C₅-C₆)-Cycloalkyl steht, und
R² für Wasserstoff, Formyl, unverzweigtes oder verweigtes (C₁-C₁₀)-Alkyl-carbonyl, das unsubstituiert oder im Alkylteil durch einen oder zwei Reste aus der Gruppe Hydroxy, Halogen, Phenyl oder Phenyl, welches durch 1 bis 3 Reste aus (C₁-C₄)-Alkyl, (C₁-C₄)-Alkoxy und Halogen substituiert ist, (C₁-C₄)-Alkoxy und (C₁-C₄)-Alkylthio substituiert ist, oder für Benzoyl oder Benzoyl, das durch 1 bis 3 Reste aus der Gruppe C₁-C₄-Alkyl, C₁-C₄-Alkoxy und Halogen substituiert ist, steht.

7. Verfahren gemäß Anspruch 1, in dem
R¹ für unverzweigtes oder verzweigtes (C₁-C₁₀)-Alkyl steht,
R² für Wasserstoff, (C₁-C₁₀)-Alkyl-carbonyl, das durch Phenyl oder durch ein- bis dreifach substituiertes Phenyl, dessen 1 bis 3 Substituenten aus (C₁-C₄)-Alkyl, (C₁-C₄)-Alkoxy und Halogen ausgewählt sind, substituiert ist, vorzugsweise Phenacetyl, oder für Benzoyl steht.

8. Verfahren gemäß Anspruch 1, in dem (C₁-C₆)-Alkyl die Reste Methyl, Ethyl, 1-Propyl oder 2-Propyl, n-, i-, tert- oder 2-Butyl, 3-Methyl-but-2-yl, n-, i-, tert-, 2- oder 3-Pentyl, n-hexyl oder ein stereoisomeres Hexyl bedeutet.

9. Verfahren gemäß Anspruch 1, in dem Halogen die Reste Fluor, Chlor, Brom oder Jod, vorzugsweise Fluor und Chlor, insbesondere Chlor bedeutet.

10. Verbindung der Formel (I) gemäß Anspruch 1.

## Claims

1. A process for the enzymatic separation of PTC derivatives, which comprises treating a mixture of D- and L-PTC derivatives of the formulae (I) and (II) 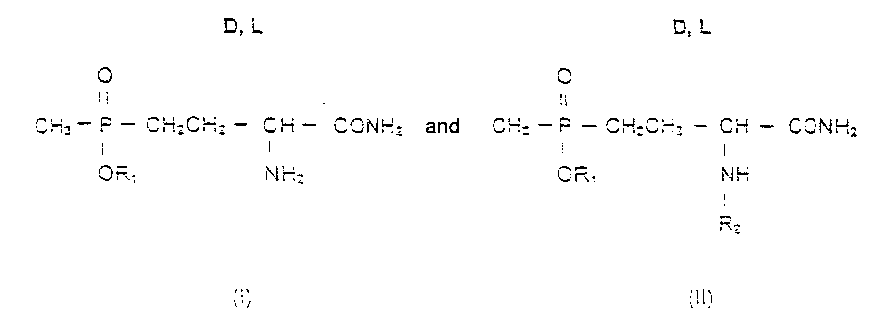 in which
R¹ is unbranched or branched (C₁-C₂₀)-alkyl which is unsubstituted or substituted by one or more halogen radicals, such as fluorine, chlorine, bromine or iodine, or mono- or polysubstituted by (C₁-C₈)-alkoxy, or is (C₃-C₈)-cycloalkyl which can be substituted by one or more groups selected from the group consisting of (C₁-C₄)-alkyl, (C₁-C₄)-alkoxy and halogen, or is (C₃-C₁₀)-alkenyl, (C₃-C₁₀)-alkynyl or benzyl, and
R² is formyl, unbranched or branched (C₁-C₂₀)-alkylcarbonyl which is unsubstituted or substituted in the alkyl moiety by one or more radicals selected from the group consisting of hydroxyl, halogen, (C₁-C₄)-alkoxy, (C₁-C₄)-alkylthio and phenyl which can be substituted by up to 3 radicals selected from the group consisting of (C₁-C₁₂)-alkyl, (C₁-C₁₂)-alkoxy, halogen, nitro and CF₃, or is benzoyl or benzoyl which is substituted by 1 to 3 radicals selected from the group consisting of (C₁-C₁₂)-alkyl, (C₁-C₁₂)-alkoxy, halogen, nitro and CF₃,
with a hydrolytically active enzyme in an aqueous or aqueous-organic medium, a selectively acting L-amino acid amide-cleaving enzyme or microorganism being used.

2. The process as claimed in claim 1, wherein a selectively acting L-amino acid amide-cleaving enzyme or a microorganism which synthesizes this enzyme is used.

3. The process as claimed in claim 1, wherein at least one microorganism selected from the group consisting of Enterobacter aerogenes (DSM 9164), Klebsiella oxytoca (DSM 9162), Klebsiella trevisanii (DSM 9163), Corynebacterium aquaticum (DSM 9171), Rhodococcus rubropertinctus (ATCC 21930), Rhodococcus rhodochrous (ATCC 33278), Arthrobacter sp. (ATCC 31652) and Corynebacterium sp. (ATCC 31662) is used.

4. The process as claimed in claim 1, wherein racemic amino acid amide derivatives, or amino acid amide derivatives whose L-form has been enriched, of the formulae (I) or (II) are employed.

5. The process as claimed in claim 1, wherein the reaction is carried out in the presence of a D-amino acid amide racemase.

6. The process as claimed in claim 1, in which
R¹ is unbranched or branched (C₁-C₁₀)-alkyl or (C₁-C₁₀)-alkyl which is substituted by halogen, such as fluorine, chlorine or by (C₁-C₄)-alkoxy, or is (C₅-C₆)-cycloalkyl, and
R² is hydrogen, formyl, unbranched or branched (C₁-C₁₀)-alkylcarbonyl which is unsubstituted or substituted in the alkyl moiety by one or two radicals selected from the group consisting of hydroxyl, halogen, (C₁-C₄)-alkoxy, (C₁-C₄)-alkylthio, phenyl or phenyl which is substituted by 1 to 3 radicals selected from the group consisting of (C₁-C₄)-alkyl, (C₁-C₄)-alkoxy and halogen, or is benzoyl or benzoyl which is substituted by 1 to 3 radicals selected from the group consisting of C₁-C₄-alkyl, C₁-C₄-alkoxy and halogen.

7. The process as claimed in claim 1, wherein
R¹ is unbranched or branched (C₁-C₁₀)-alkyl and
R² is hydrogen, (C₁-C₁₀)-alkylcarbonyl which is substituted by phenyl or by phenyl which is mono- to trisubstituted and whose 1 to 3 substituents are selected from the group consisting of (C₁-C₄)-alkyl, (C₁-C₄)-alkoxy and halogen, preferably phenacetyl, or is benzoyl.

8. The process as claimed in claim 1, wherein (C₁-C₆)-alkyl is a radical methyl, ethyl, 1-propyl or 2-propyl, n-, i-, tert- or 2-butyl, 3-methyl-but-2-yl, n-, i-, tert-, 2- or 3-pentyl, n-hexyl or a stereoisomeric hexyl.

9. The process as claimed in claim 1, wherein halogen is a radical fluorine, chlorine, bromine or iodine, preferably fluorine or chlorine, in particular chlorine.

10. A compound of the formula (I) as claimed in claim 1.

## Revendications

1. Procédé pour la séparation enzymatique de dérivés de PTC, **caractérisé en ce qu'**on traite un mélange de dérivés de D- et de L-PTC de formules générales (I) et (II) 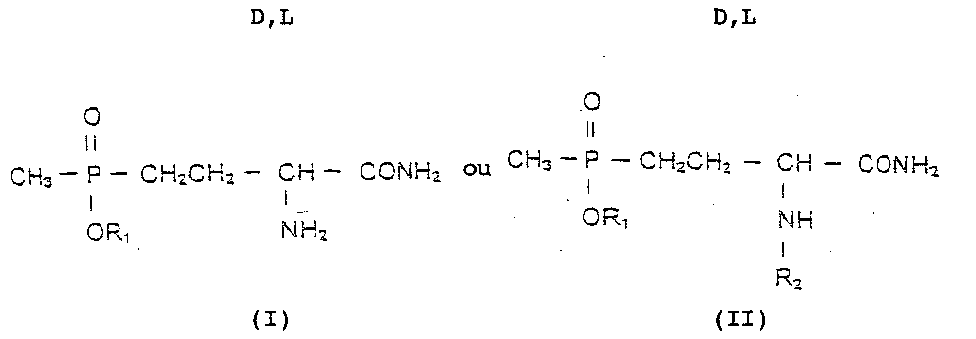 dans lesquelles
R¹ représente un groupe alkyle en C₁-C₂₀ à chaîne droite ou ramifiée qui est non substitué ou substitué par un ou plusieurs restes halogène, tels que les restes fluor, chlore, brome ou iode, ou substitué une ou plusieurs fois par un substituant alkoxy en C₁-C₈, ou représente un groupe cycloalkyle en C₃-C₈ qui peut être substitué par un ou plusieurs groupes alkyle en C₁-C₄, alkoxy en C₁-C₄ et halogène, ou représente des groupes alcényle en C₃-C₁₀, alcynyle en C₃-C₁₀ ou benzyle, et
R² représente des groupes formyle, (alkyl en C₁-C₂₀)-carbonyle à chaîne droite ou ramifiée, qui est non substitué ou substitué dans le fragment alkyle par un ou plusieurs restes pris parmi les restes hydroxy, halogène, phényle qui peut être substitué par jusqu'à 3 restes pris dans le groupe comportant alkyle en C₁-C₁₂, alkoxy en C₁-C₁₂, halogène, nitro et CF₃, qui est substitué par des restes alkoxy en C₁-C₄ et (alkyl en C₁-C₄)thio, représente benzoyle ou benzoyle qui est substitué par 1 à 3 restes pris dans le groupe comportant alkyle en C₁-C₁₂, alkoxy en C₁-C₁₂, halogène, nitro et CF₃,
en milieu aqueux ou aqueux-organique, par une enzyme à activité hydrolytique, en utilisant une enzyme ou un microorganisme présentant une action sélective de clivage des amides de L-aminoacides.

2. Procédé selon la revendication 1, dans lequel on utilise une enzyme ou un microorganisme qui synthétise cette enzyme présentant une activité sélective de clivage des amides de L-aminoacides.

3. Procédé selon la revendication 1, dans lequel on utilise au moins un microorganisme pris dans le groupe qui est constitué par *Enterobacter aerogenes* (DSM 9164), *Klebsiella oxytoca* (DSM 9162), *Klebsiella trevisanii* (DSM 9163), *Corynebacterium aquaticum* (DSM 9171), *Rhodococcus rubropertinctus* (ATCC 21930), *Rhodococcus rhodochrous* (ATCC 33278), *Arthrobacter sp.* (ATCC 31652) et *Corynebacterium sp.* (ATCC 31662).

4. Procédé selon la revendication 1, dans lequel on utilise des dérivés d'amides d'aminoacides de formules générales (I) ou (II) racémiques ou enrichis en forme L.

5. Procédé selon la revendication 1, dans lequel on met en oeuvre la réaction en présence d'une racémase d'amides de D-aminoacides.

6. Procédé selon la revendication 1, dans lequel
R¹ représente un groupe alkyle en C₁-C₁₀ à chaîne droite ou ramifiée ou un groupe alkyle en C₁-C₁₀ qui est substitué par des substituants halogène comme fluor, chlore, ou par alkoxy en C₁-C₄, ou représente un groupe cycloalkyle en C₅-C₆, et
R² représente un atome d'hydrogène, des groupes formyle, (alkyl en C₁-C₁₀)-carbonyle à chaîne droite ou ramifiée, qui est non substitué ou substitué dans le fragment alkyle par un ou deux restes pris dans le groupe des restes hydroxy, halogène, phényle ou phényle substitué 1 à 3 restes pris parmi alkyle en C₁-C₄, alkoxy en C₁-C₄ et halogène, est substitué par alkoxy en C₁-C₄ et (alkyl en C₁-C₄)thio, ou représente benzoyle ou benzoyle qui est substitué par 1 à 3 restes pris dans le groupe comportant alkyle en C₁-C₄, alkoxy en C₁-C₄ et halogène.

7. Procédé selon la revendication 1, dans lequel
R¹ représente un groupe alkyle en C₁-C₁₀ à chaîne droite ou ramifiée, et
R² représente un atome d'hydrogène, des groupes (alkyl en C₁-C₁₀)-carbonyle qui est substitué par un substituant phényle ou phényle substitué une à trois fois, dont 1 à 3 substituants sont pris parmi alkyle en C₁-C₄, alkoxy en C₁-C₄ et halogène, de préférence phénacétyle, ou représente benzoyle.

8. Procédé selon la revendication 1, dans lequel le groupe alkyle en C₁-C₆ représente les restes méthyle, éthyle, 1-propyle ou 2-propyle, n-, i-, tert- ou 2-butyle, 3-méthyle-but-2-yle, n-, i-, tert-, 2- ou 3-pentyle, n-hexyle ou un hexyle stéréoisomère.

9. Procédé selon la revendication 1 dans lequel halogène représente les restes fluor, chlore, brome ou iode, de préférence fluor et chlore, notamment chlore.

10. Composé de formule (I) selon la revendication 1.
